# EUROPEAN PATENT SPECIFICATION

(11) **EP 3 191 146 B1**
(45) Date of publication and mention of the grant of the patent: **07.11.2018**
(21) Application number: 15760474.5
(22) Date of filing: 10.09.2015
(51) Int. Cl.: A61L 27/30, A61N 1/18

(54) **IMPLANTABLE ELECTRODE**
IMPLANTIERBARE ELEKTRODE
ÉLECTRODE IMPLANTABLE

(30) Priority: 10.09.2014 LU 92540
(43) Date of publication of application: 19.07.2017
(73) Proprietor: Luxembourg Institute of Science and Technology (LIST), 4362 Esch-sur-Alzette (LU)
(72) Inventor: LENOBLE, Damien, B-69200 Wellin (BE); THOMANN, Jean-Sébastien, F-54730 Gorcy (FR); PALISSOT, Valérie, F-57100 Thionville (FR)
(74) Representative: Lecomte & Partners
(86) International application number: PCT/EP2015/070759
(87) International publication number: WO 2016/038161

(56) References cited:
- WO-A1-2009/065171
- ELIAS ESTEPHAN ET AL: "Phages recognizing the Indium Nitride semiconductor surface via their peptides", JOURNAL OF PEPTIDE SCIENCE, vol. 17, no. 2, 30 November 2010 (2010-11-30), pages 143-147, XP055187050, ISSN: 1075-2617, DOI: 10.1002/psc.1315

## Description

### Technical field

The present invention relates to the field of implantable materials and devices. In particular it relates to an electrode for carrying electrical signals to and/or from biological cells or tissues which are directly contacted by the electrode.

### Background of the invention

Several materials and devices have been investigated and proposed to mimic, probe or/and restore activities of different biological tissues and cells. Biocompatible electrodes for cell stimulation or cell monitoring are still needed as a building block for devices acting as brain mechanical interfaces. As an example, the first successful neuroprosthetics-cochlear implant put to practice in the early 1990s is a neural interface device that uses an electrode array to stimulate the auditory nerve in the cochlea with electric impulses. Electrical stimulation of peripheral nerves has gained a lot of interest from clinicians for the treatment of pain, for restoration of motricity functions and for the treatment of epilepsy. Additionally, the neuroengineering research community has investigated various neural interface devices for the Central Nervous System, CNS. Such devices are designed to regulate mood disorders, epilepsy or symptoms arising with Parkinson's disease, through appropriate electric charge deliveries with deep brain stimulation. Recording of brain signals has been used to control a brain-computer interface, BCI [1].

Implantable devices and neural interfaces in particular face several challenges:
- reduce the inflammatory response and gliosis around implants, as these reduce the signal to noise ratio delivered to/from the devices or lead to chronic recording failures [2];
- improve the spatial resolution of neural interfaces that allows the collection and the delivery of signals to or from a portion of the neuron-dense brain;
- selectively stimulate precise areas to induce specific responses from the CNS.

In what follows and throughout the description of the present application, the term biocompatibility will be used to refer to the ability of an implanted device and its component materials to perform their desired function in their implanted environment, maintaining beneficial cellular and tissue responses, and not eliciting undesirable local or systemic effects in the body.

Further, bio-fouling of an implanted device or material refers to the uncontrolled growth of tissue on the device or material.

While several materials have been tested for in vivo bio implant applications, polymers are nowadays the materials of choice due to their plasticity and biocompatibility. In brain-mechanical interfaces, BMI, polymers are mostly used as encapsulation matrices for semiconducting materials. Several polymers, such as poly(3,4-ethylenedioxythiophene), PEDOT, polyaniline, or polythiophene are known to have conductive properties. The use of conductive polymers can increase the biocompatibility of an implanted material and enable interaction with surrounding cells. However, conductive polymers remain very sensitive to changes in impedance caused by bio-fouling or the uptake of the materials in resident immune cells of the brain [3].

Estephan et al. have discussed the biocompatible properties of Indium Nitride in "Phages recognizing the Indium Nitride semiconductor surface via their peptides", J. Pept. Sci. 2011; 17: 143-147.

Patent document WO 2009/065171 A1 discloses an electrode array for a cochlear implant. The use of silicon to prevent adhesion of cells or tissue to the implant is discussed therein.

### Technical problem to be solved

It is therefore an objective of the present invention to provide an implantable electrode for carrying an electric signal from an implanted device to biological cells or from biological cells to an implanted device, which overcomes at least some of the disadvantages of the prior art.

### Summary of the invention

According to a first aspect of the invention, an implantable electrode for carrying an electrical signal is provided. The electrode comprises at least one portion for directly contacting a biological cell or tissue. The electrode is remarkable in that said portion is made of Indium nitride, InN, so that said portion is electrically conductive or semiconductive and so that cell adhesion on said portion is inhibited.

The surface of the first portion may comprise a thin layer having impurities such as for example Indium tri-oxyde, In₂O₃, or traces of hydrogen.

Preferably, the electrode may be entirely made of Indium nitride, InN.

The electrode portion may preferably comprise a coating of said electrode. The coating may more preferably integrally cover said electrode.

The electrode portion may preferably comprise connection means for electrically connecting the electrode to a biological cell or tissue. The connection means may preferably comprise at least one electrode end of said electrode. In particular, the electrode may be wire-shaped.

Preferably, the electrode may comprise at least one portion, which is distinct of said aforementioned portion made of InN, and wherein said second portion is made of a conducting or semiconducting material.

The electrode may preferably have a layered structure comprising a top layer, which comprises said portion made of InN for directly contacting a biological cell or tissue.

According to a further aspect of the invention, there is provided an implantable device. The implantable device comprises at least one electrode for carrying an electrical signal, which conforms to the electrode according to the invention. Preferably, the device is an electronic device.

The device may further comprise signal processing means, which are operatively connected to said electrode and which are configured for processing a signal carried by said electrode.

According to yet another aspect of the invention, the use of Indium nitride, InN, as an implantable electrode material for contacting a biological cell or tissue, for carrying an electric signal and for inhibiting cell adhesion on the electrode is provided.

The implantable electrode in accordance with the invention may be used for transmitting an electrical signal either to or from a biological cell or tissue, with which the implanted electrode is put into direct contact. At least one portion of the electrode, which directly contacts the biological tissue into which the electrode is implanted, is made of Indium nitride, InN. It has been observed that InN regroups in one material several desired properties for implantable electrodes. Namely it is electrically conductive (or semiconductive), it inhibits cell adhesion and therefore it inhibits cell growth on its surface even if it is directly contacted by cells over a long time period. Furthermore, it exhibits no toxicity to the biological environment into which it is implanted.

Inhibition of cell adhesion and cell growth on InN means that these phenomena are prevented or significantly slowed down, depending on the cells that are contacted by InN. As cell growth is inhibited on InN, the phenomenon of bio-fouling described above is reduced as compared to known implantable electrode materials. The effect of reduced bio-fouling or bio-adhesion is an observed improvement of the signal to noise ratio that is measurable for electrical signals that are carried by the electrode. Indeed, once an appropriate contact has been established between the electrode and the cell/tissue during implantation, the quality of the contact is not impaired by bio-fouling. The longevity of the implant is therefore enhanced, which reduces the need for maintenance of the electrode and/or repeated surgery for the implanted person.

While polymer coatings have been suggested to improve on the biocompatibility of known implantable conductive electrodes, the use of InN electrode eliminates the need for a specific coating, as the electrode material itself exhibits biocompatibility with the cells/tissue that are directly contacted by the electrode. An implantable electrode in accordance with the present invention is therefore easier to produce and to maintain as known implantable electrodes made of other materials.

As the use of an InN electrode eliminates the need for a polymer coating, issues relating to the toxicity of polymers are alleviated. Toxicity levels of InN have been found to be very low.

### Brief description of the drawings

Several embodiments of the present invention are illustrated by way of figures, which do not limit the scope of the invention, wherein:
- figures 1a and 1b schematically illustrate the structure of samples having a GaN and InN top layer respectively;
- figure 2a and 2b illustrate cell-viability results obtained using the samples of Figure 1 for the cell lines MCF10A and HS-5 respectively;
- figures 3a and 3b illustrate cell adhesion results obtained using the samples of Figure 1, wherein adhesion of different cell lines on the GaN and InN samples are shown in Figure 3a and 3b respectively;
- figure 4 schematically illustrates a preferred embodiment of the implantable electrode according to the invention;
- figure 5 schematically illustrates a preferred embodiment of the implantable electrode according to the invention;
- figure 6 schematically illustrates a preferred embodiment of the implantable electrode according to the invention;
- figure 7 schematically illustrates a preferred embodiment of the implantable electrode according to the invention;
- figure 8 schematically illustrates a cut view of a preferred embodiment of the implantable electrode according to the invention;
- figure 9 schematically illustrates a cut view of a preferred embodiment of the implantable electrode according to the invention;
- figure 10 schematically illustrates a preferred embodiment of an implantable device according to the invention.

### Detailed description of the invention

This section describes the invention in further detail based on preferred embodiments and on the figures. It should be understood that technical features presented for a specific embodiment may be combined with features of other embodiments, unless specifically noted otherwise. First, the materials and methods used for obtaining the presented results are described.

### Materials and methods

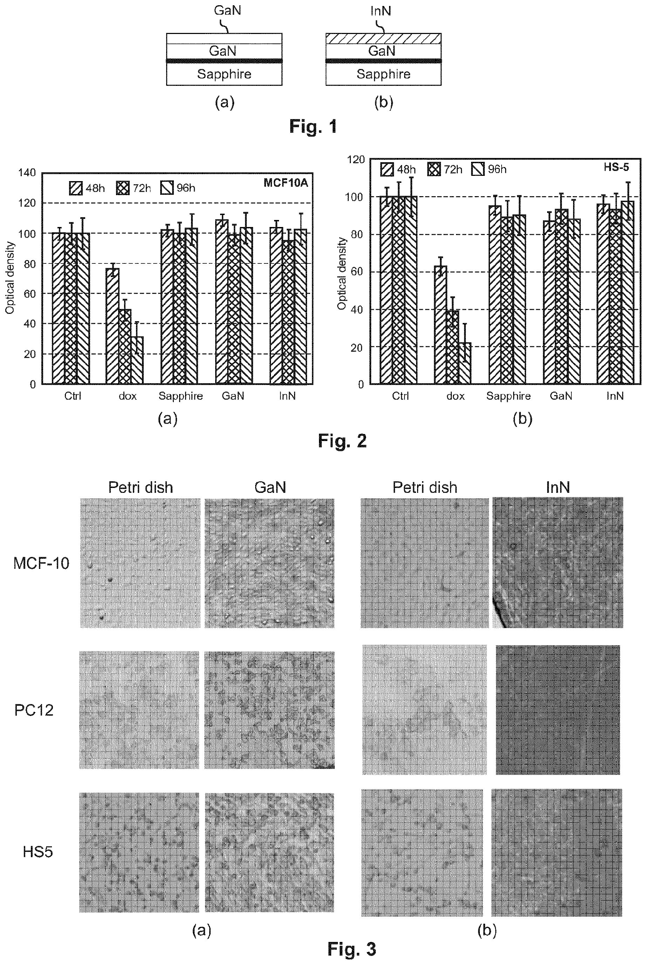

To simulate cell behavior in the presence of a device comprising a top layer, i.e. a cell-contacting layer, comprising Indium nitride InN material, different structures were synthesized by using Chemical Vapor Deposition, CVD, on Sapphire or silicon substrate. The structures shown in Figures 1a and 1b comprise a bottom insulating layer of Sapphire, which acts as a substrate for several layers which form an electrode. The top layer is the only layer which is directly contacted with biological cells during tests. Figure 1a shows a cell-contacting top layer made of GaN, while Figure 1b shows a cell-contacting layer made of InN. The top layer has a thickness of about 200 nm. The illustrated intermediate layers were chosen in order to optimize the transport of electrical signals to and from the GaN respectively InN top layers.

Their presence does not affect the results as to bio-adhesion and toxicity of the top layers. Cell-adhesion and -growth on Gallium nitride is known from the prior art. In order to assess the toxicity and the bio-adhesion of cells in presence of the respective top layer, Sigma-Aldrich XTT™ tests and bio-adhesion studies have been performed.

The experimental setup used for obtaining the reported results is described in what follows.

In order to perform adhesion microscopy, elements were placed in 24-well plates with 25 mm² elements per well. 500 µl of GFP(green fluorescent protein)-PC12 suspension containing 5'000 and 10'000 cells respectively in Dulbecco's Modified Eagle's Medium, DMEM -1% horse serum with nerve growth factor 100ng/ml was added and incubated for 72 hours.

Before imaging on a Westburg Evos™ microscope, supernatants were aspirated and replaced with fresh DMEM medium without phenol red. Imaging of the elements was performed either by confocal microscopy experiments using a Zeiss LSM 510™ in fresh DMEM medium without phenol red, or by optical microscopy in reflective mode using a Zeiss™ microscope in phosphate-buffered saline, PBS.

Pfizer Adriblastina™ was used as Doxorubicin, dox, ready-to-use injectable solution. Invitrogen™ NGF2.5S nerve growth factor was diluted in PBS/0.1% bovine serumalbumin and stored at -20°C.

Three adherent cell lines were used in tests. First, the human mammary non-tumorigenic epithelial cell line MCF10A was grown in DMEM/F12 medium supplemented with 5% horse serum, epidermal growth factor EGF (Sigma-Aldrich™ 20ng/ml), hydrocortisone (Sigma-Aldrich™ 0.5 mg/ml), cholera toxin (Gentaur™ 100ng/ml), insulin (Sigma-Aldrich™ 10mg/ml), 100U/ml of penicillin and streptomycin.

Second, the HS-5 stromal cell line was cultivated in DMEM, 10% fetal bovine serum, 100U/ml of penicillin and streptomycin.

Third, the PC-12 cell line derived from a transplantable rat neuroendocrine tumor of the adrenal medulla was grown in 10% horse serum, 5% fetal bovine serum, 100U/ml of penicillin and streptomycin. Neuronal phenotype on this model is induced using nerve growth factor 100 ng/ml in DMEM -1% horse serum for 72 hours. PC12 cells stop dividing and terminally differentiate when treated with nerve growth factor. This makes PC12 cells useful as a model system for neuronal differentiation.

All cell lines were maintained at a temperature of 37°C in an ambient environment containing 5% CO₂ and 95% humidity.

Cell viability was determined using a colorimetric Sigma-Aldrich XTT™ assay in accordance with the manufacturer's instructions. 3'000 cells per well were plated on a 48-well plate with or without the different elements for 46 to 96 hours. Four hours before the end of the exposure, XTT was added. At the end of the exposure, optical densities of supernatants were read at 490 nm. A positive control of toxicity was obtained with doxorubicin treatment.

The results obtained for the described samples using the described methods and experimental setups are illustrated in Figures 2 and 3.

Figures 2a and 2b illustrate the viability of MCF10 mammary epithelial cells (in Figure 2a) and HS5 stromal cells (in Figure 2b) on a respective cell-contacting layer of Sapphire, GaN and InN. Cells were incubated for 48, 72 or 96 hours in presence of the different samples, including a control and dox sample. The latter is a positive control, which induces cell death. Results are expressed in percentage (Optical Density, OD, calculated for a given sample / OD calculated for the control sample) and statistical errors are indicated per result. As can be observed, the HS5 of MCF10 cell viability is not affected in the presence of InN and Sapphire. The positive Dox control proves that the tested cells are not over-resistant.

Figures 3a and 3b illustrate the cell bio-adhesion results obtained as described on the different cell-contacting layers made of GaN for Figure 3a and of InN for Figure 3b. Besides HS5 and MCF10 cells, PC12 cells were tested as a model for neuronal cells. In the case of InN, substantially no cells are able to adhere on the substrate. In the petri dish, cells have grown on the plastic part as in the control sample, with no sign of induced toxicity. This confirms the viability results shown in Figures 2a and 2b.

In all described embodiments, the surface of the Indium nitride, InN, layer may comprise a thin layer having impurities such as for example Indium tri-oxyde, In₂O₃, or traces of hydrogen.

### Applications:

Several applications can be realized based on the surprising effect of inhibited bio-fouling on Indium nitrate electrodes, of which some are described by way of examples only, without limiting the scope of the present invention thereto.

In a preferred embodiment of the invention, an implantable device comprises an implantable electrode. The electrode comprises at least one portion for directly contacting a biological cell or tissue. The portion is made of InN and allows for carrying an electrical signal to or from the biological cell or tissue. In particular, the electrode can be used to probe/sense cell activity or to deliver electric impulses to the cell or tissue. The electrode may also be used to provide an electrical connection between two cells or tissue regions, wherein each region is directly contacted by a specific InN portion of the electrode.

In the following embodiments, similar concepts will be referred to by similar reference numbers. For example, reference numbers 10, 20, 30, ... will be used to identify an electrode of an implantable device in accordance with the invention across distinct embodiments.

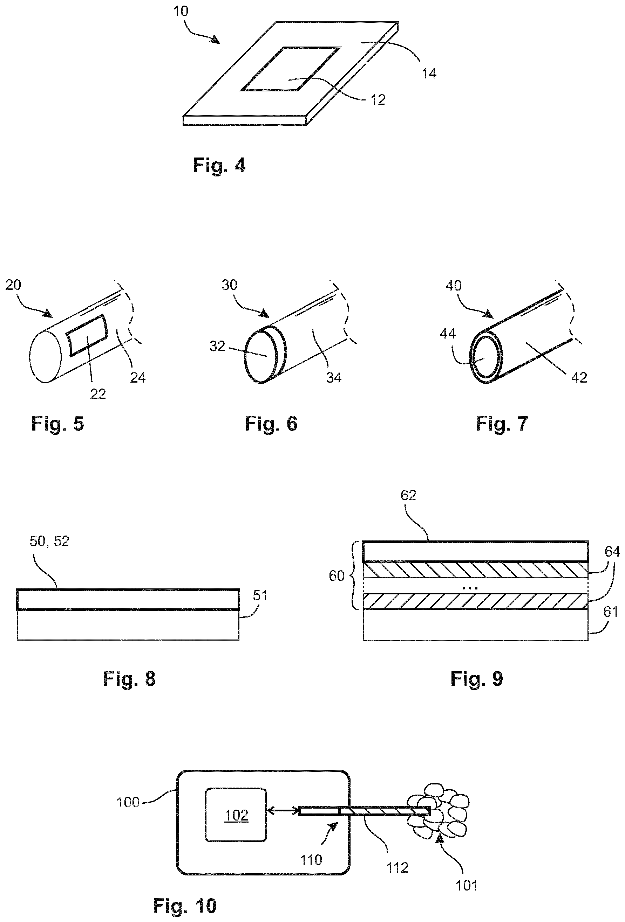

Figure 4 shows an embodiment of the electrode 10 of an implantable device according to the invention. For the sake of clarity, only the electrode 10 is depicted. By way of example a planar electrode is shown, comprising a portion 12 made of InN, which is used to contact biological cells or tissue. In alternative embodiments, the electrode may be arbitrarily shaped and comprise a plurality of portions 12 made of InN. As shown, the electrode 10 may comprise at least one further portion 14, which is not made of InN, but of another electrically conducting or semiconducting material. As the portion 14 is not used for directly contacting biological cells or tissue, the toxicity and bio-fouling requirements of portion 14 are less strict than those of portion 12. The material or materials of portion 14 may depend on the particular application in which the InN portion 12 is used. The skilled person will be able to select suitable materials for portion 14 to optimize, for example, the transport of electrical signals to and from the portion 12.

In the embodiment shown in Figure 5, the electrode 20 is a wire-shaped electrode comprising a surface portion 22 made of InN, which is suitable for directly and durably contacting biological cells or tissue as it inhibits bio-fouling thereon and exhibits low toxicity with regard to the contacted cells.

In the embodiment shown in Figure 6, the electrode 30 is a wire-shaped electrode comprising a surface portion 32 made of InN, which covers an electrode end. The electrode end is therefore made suitable for directly and durably contacting biological cells or tissue as it inhibits bio-fouling thereon and exhibits low toxicity with regard to the contacted cells.

According to another embodiment of the invention, illustrated in Figure 7, the portion 42 made of InN is a coating of the implantable electrode 40, which preferably covers the entire electrode. As compared to known polymer coatings which are also used for providing a biocompatible surface, an InN coating 42 has the additional advantage that it exhibits reduced toxicity in relation to the surrounding cells or tissue.

As shown in the embodiment Figure 8, the portion 52 may comprise the entire electrode 50. In the example shown, the bulk InN electrode 50 is supported on a substrate 51, which may be a conducting or insulating substrate depending on the application in which the electrode 50 is used.

Figure 9 illustrates a further electrode 60 of an implantable device according to the invention, which is supported by substrate 61. Apart from the top layer 62, which is suitable for directly and durably contacting biological cells or tissue, and to transport electrical signals therefrom and/or thereto, the electrode 60 further comprises one or more lower layers 64, which are not suitable for directly contacting biological cells or tissue.

From the above results and description, it will be apparent to the skilled person that an electrode according to the invention is an implantable electrode, which enables to establish durable electrical connections between an implanted device and a tissue into which the device has been implanted, between such devices, or between such tissues. As a result of the good compatibility with the PC12 cell lines, the electrode according to the invention finds particular use in Brain-Computer interface applications, for which the selective stimulation/probing of precise areas of the brain is necessary. The described electrode portions comprising InN may be produced by known methods such as CVD or others, which will be within the reach of the skilled person, and the description of which would extend beyond the context of the present application. The InN portions, layers or coatings may be produced as thick layers or thin layers, preferably of micro- or nano-scale dimensions. In particular, the electrode may be provided as part of a printed circuit on a substrate.

According to a further embodiment of the invention, an implantable electronic device 100 is provided, which comprises at least one electrode 110 comprising at least one portion 112 made of InN for directly contacting a biological cell or tissue 101. The implantable device comprises signal processing means 102 which are as such known by the person skilled in the art and which will not be further detailed in the context of the present description. The signal processing means may comprise means for amplifying or filtering a received signal. The signal processing means are operatively connected to the electrode which directly contacts the biological tissue.
The electrode 110 may therefore be used to transmit a signal emitted by the cell or tissue to the signal processing means 102 or to transmit a processed signal to the cell or tissue. In the latter case, the implantable device may further comprise not illustrated signal generating means such as a tunable voltage or current source and appropriate controlling/processing means as an input to the signal processing path. Such means are as such known to the skilled person.
The implantable device may further comprise functionalized components or semiconductors such as MOSFETs for processing or transmitting a received signal to or from the implantable device. The described components are preferably provided on a common substrate of the implantable device.
It should be understood that the detailed description of specific preferred embodiments is given by way of illustration only, since various changes and modifications within the scope of the invention will be apparent to the skilled man. The scope of protection is defined by the following set of claims.

### References

[1] Santhanam G, Ryu SI, Yu BM, Afshar A, Shenoy KV, Nature 442:195-98 (2006)
[2] Turner JN, Shain W, Szarowski DH, Andersen M, Martins S, et al., Exp. Neurol. 156:33-49 (1999)
[3] Bellamkonda, R. V.; Pai, S. B.; Renaud, P. MRS Bulletin 2012, 37 (06), 557-561.

## Claims

1. An implantable device (100) comprising at least one electrode (10, 20, 30, 40, 50, 60, 110) for carrying an electrical signal, **characterized in that** said electrode comprises at least one portion (12, 22, 32, 42, 52, 62) for directly contacting a biological cell or tissue, wherein said portion (12, 22, 32, 42, 52, 62) is made of Indium nitride, InN, so that said portion is electrically conductive or semiconductive and so that cell adhesion on said portion is inhibited.

2. The device of claim 1, wherein the electrode is entirely made of Indium nitride, InN.

3. The device of claim 1, wherein said at least one electrode portion (42) comprises a coating of said electrode.

4. The device of claim 3, wherein said coating integrally covers said electrode.

5. The device according to any of claims 1 to 4, wherein said electrode portion comprises connection means for electrically connecting the electrode to a biological cell or tissue.

6. The device of claim 5, wherein said connection means comprise at least one electrode end 32 of said electrode.

7. The device according to any of claims 1 to 6, wherein the electrode comprises at least one portion (14, 24, 34, 44, 54, 64), distinct of said portion made of InN (12, 22, 32, 42, 52, 62), and wherein said portion (12, 22, 32, 42, 52, 62) is made of a conducting or semiconducting material.

8. The device according to any of claims 1 to 7, wherein the electrode has a layered structure comprising a top layer, which comprises said portion (14, 24, 34, 44, 64) for directly contacting a biological cell or tissue.

9. The device according to any of claims 1 to 8, further comprising signal processing means (102) operatively connected to said electrode (110) and configured for processing a signal carried by said electrode.

10. The use of Indium nitride, InN, as an implantable electrode material for contacting a biological cell or tissue, for carrying an electric signal and for inhibiting cell adhesion on the electrode.

## Patentansprüche

1. Implantierbare Vorrichtung (100), umfassend mindestens eine Elektrode (10, 20, 30, 40, 50, 60, 110) zum Befördern eines elektrischen Signals, **dadurch gekennzeichnet, dass** die Elektrode mindestens einen Abschnitt (12, 22, 32, 42, 52, 62) zum direkten Kontaktieren einer biologischen Zelle oder Gewebes umfasst, wobei besagter Abschnitt (12,22,32,42,52,62) aus Indiumnitrid, InN, hergestellt ist, sodass besagter Abschnitt elektrisch leitend oder halbleitend ist und sodass Zelladhäsion auf diesem Abschnitt inhibiert wird.

2. Vorrichtung nach Anspruch 1, wobei die Elektrode vollständig aus Indiumnitrid, InN, hergestellt ist.

3. Vorrichtung nach Anspruch 1, wobei der mindestens eine Elektrodenabschnitt (42) eine Beschichtung dieser Elektrode umfasst.

4. Vorrichtung nach Anspruch 3, wobei die Beschichtung die Elektrode integral bedeckt.

5. Vorrichtung nach einem der Ansprüche 1 bis 4, wobei der Elektrodenabschnitt Verbindungsmittel zum elektrischen Verbinden der Elektrode mit einer biologischen Zelle oder Gewebe umfasst.

6. Vorrichtung nach Anspruch 5, wobei die Verbindungsmittel mindestens ein Elektrodenende 32 der Elektrode umfassen.

7. Vorrichtung nach einem der Ansprüche 1 bis 6, wobei die Elektrode mindestens einen Abschnitt (14, 24, 34, 44, 54, 64), verschieden von dem besagten aus InN hergestellten Abschnitt (12,22,32,42,52,62), umfasst und wobei besagter Abschnitt (12,22,32,42,52,62) aus einem leitenden oder halbleitenden Material hergestellt ist.

8. Vorrichtung nach einem der Ansprüche 1 bis 7, wobei die Elektrode eine Schichtstruktur aufweist, die eine Decklage umfasst, die den Abschnitt (14,24,34,44,64) zum direkten Kontaktieren einer biologischen Zelle oder Gewebes umfasst.

9. Vorrichtung nach einem der Ansprüche 1 bis 8, weiter Signalverarbeitungsmittel (102) umfassend, die wirksam mit der Elektrode (110) verbunden und dafür ausgelegt sind, ein von der Elektrode befördertes Signal zu verarbeiten.

10. Anwendung von Indiumnitrid, InN, als implantierbares Elektrodenmaterial zum Kontaktieren einer biologischen Zelle oder Gewebes, zum Befördern eines elektrischen Signals und zum Inhibieren von Zelladhäsion an der Elektrode.

## Revendications

1. Dispositif implantable (100) comprenant au moins une électrode (10, 20, 30, 40, 50, 60, 110) destinée à acheminer un signal électrique, **caractérisé en ce que** ladite électrode comprend au moins une portion (12, 22, 32, 42, 52, 62) pour une mise en contact directe avec un tissu ou une cellule biologique ; dans lequel ladite portion (12, 22, 32, 42, 52, 62) est constituée de nitrure d'indium, InN, d'une manière telle que ladite portion est électroconductrice ou semi-conductrice et d'une manière telle que l'on inhibe l'adhérence cellulaire sur ladite portion.

2. Dispositif selon la revendication 1, dans lequel l'électrode est entièrement constituée de nitrure d'indium, InN.

3. Dispositif selon la revendication 1, dans lequel ladite au moins une portion d'électrode (42) comprend un revêtement de ladite électrode.

4. Dispositif selon la revendication 3, dans lequel ledit revêtement recouvre intégralement ladite électrode.

5. Dispositif selon l'une quelconque des revendications 1 à 4, dans lequel ladite portion d'électrode comprend des moyens de liaison pour relier par voie électrique l'électrode à un tissu ou une cellule biologique.

6. Dispositif selon la revendication 5, dans lequel lesdits moyens de liaison comprennent au moins une extrémité d'électrode (32) de ladite électrode.

7. Dispositif selon l'une quelconque des revendications 1 à 6, dans lequel l'électrode comprend au moins une portion (14, 24, 34, 44, 54, 64) distincte de ladite portion constituée de InN (12, 22, 32, 42, 52, 62), et dans lequel ladite portion (12, 22, 32, 42, 52, 62) est réalisée à partir d'un matériau conducteur ou semi-conducteur.

8. Dispositif selon l'une quelconque des revendications 1 à 7, dans lequel l'électrode possède une structure stratifiée comprenant une couche supérieure, qui comprend ladite portion (14, 24, 34, 44, 54, 64) pour une mise en contact directe avec un tissu ou une cellule biologique.

9. Dispositif selon l'une quelconque des revendications 1 à 8, comprenant en outre un moyen de traitement de signaux (102) relié de manière opérationnelle à ladite électrode (110) et configuré pour traiter un signal acheminé par ladite électrode.

10. Utilisation de nitrure d'indium, InN, à titre de matière d'une électrode implantable pour la mise en contact avec un tissu ou une cellule biologique, pour l'acheminement d'un signal électrique et pour l'inhibition d'une adhérence cellulaire sur l'électrode.
